# EUROPEAN PATENT APPLICATION

(11) **EP 2 662 454 A1**
(43) Date of publication of application: **13.11.2013**
(21) Application number: 13166655.4
(22) Date of filing: 06.05.2013
(51) Int. Cl.: C12Q 1/68

(54) **Diagnostic method of pancreas cancer based on the determination of mutations of K-RAS gene**

(30) Priority: 07.05.2012 IT RM20120196
(71) Applicant: Universita' Campus Bio-Medico di Roma, 00128 Rome (IT)
(72) Inventor: Perrone, Giuseppe, 00128 Roma RM (IT); Onetti Muda, Andrea, 00128 Roma RM (IT)
(74) Representative: Germinario, Claudio

(57) **Abstract**

The present invention is directed to a method of molecular biology and a kit for the diagnosis of carcinoma of the pancreas on cytological material. The method of the present invention provides the evaluation of genetic mutations on the Kras gene by pyrosequencing. The application of this invention enables to determine with greater accuracy and diagnostic rapidity the singling out of patients suffering from pancreatic carcinoma.

## Description

The present invention is directed to a method of molecular biology and a kit for the diagnosis of carcinoma of the pancreas on cytological material. The method of the present invention provides the evaluation of genetic mutations on the Kras gene by suitable technology. The application of this invention enables to single out with greater accuracy and diagnostic rapidity patients suffering from pancreatic carcinoma.

### STATE OF THE PRIOR ART

5-year survival of patients suffering from ductal carcinoma of the pancreas (PDC) is lower than 3.5%. In 90% of cases neoplasia is diagnosed in an advanced stage and, therefore, is not surgically removable. Patients with a diagnosis of resectable tumour (in a non-advanced stage) have 5-year survival rates higher than 20%, indicating that a diagnosis in the initial stages of the disease and subsequent surgery significantly improves survival for this pathology.

With the introduction of the endoscopic ultrasound-guided fine-needle aspiration biopsy (EUS-FNA) technique, today it is possible to collect cells from suspect pancreatic lesions enabling a microscopic analysis thereof (to date, the standard technique for cytological diagnosis of adenocarcinoma of the pancreas).

Cytological examination of samples obtained with this technique enables to accurately define a sizeable share of pancreatic lesions; even so, one of the most relevant issues in the cytological diagnostics of solid lesions of the pancreas lies however in the differential diagnosis between lesions of inflammatory nature (chronic pancreatitis) and well-differentiated PDCs. Literature data *(*Turner et al Gastrointest. Endosc. 2010;71:91-8 *-* Bournet et al. Endoscopy 2009;41:552-7*)* maintain that in about 20% of the cases of solid lesions of the pancreas it is not possible to define by cytological examination the benign or malignant nature of the disease. For these patients further in-depth diagnostic analyses are required, in fact delaying PDC diagnosis.

Scientific literature data suggest the tumoral progression leading to PDC to entail somatic alterations in characteristic oncogenes. In particular, oncogene KRAS (K-ras) is critical for the start of tumorigenesis processes and found mutated in 75-90% of pancreatic carcinomas.

In the article by Tada M et al. (The American Journal of Gastroenterology 2002) a study is described in which some samples, obtained by EUS-FNA, have been subjected to cytological examinations for the diagnosis of ductal carcinoma of the pancreas and genetic analysis by semiquantitative PCR of codon 12 of the Kras gene.

In the article by Bournet et al. (Endoscopy 2009) another study is described, on a larger number of samples, always obtained by EUS-FNA. In this study, the samples were subjected to cytological examination for the diagnosis of ductal carcinoma of the pancreas and genetic analysis by RFLP PCR and direct sequencing of Kras gene codon 12 and 13. The authors conclude that analysis of Kras gene codon 12 and 13 mutation does not improve the overall diagnostic accuracy in identifying patients suffering from PDC, compared to the sole cytological analysis.

None of the diagnostic techniques proposed, based on molecular biology approaches, found application in everyday clinical practice. Attempts described in the scientific literature about the evaluation of Kras gene mutations as neoplastic pathology markers are partly conflicting, in fact not leading to a real application of such methods. Previous experiences showed that Kras gene evaluation in fact does not enable to achieve advantageous results in the diagnosis of carcinoma of the pancreas, compared to the sole morphological evaluation. These results were obtained by using cytological material derived from pancreatic lesions without an evaluation of the sample to be examined. Such material is normally divided into two parts: a) one part (the greater one) was used for morphological examination, b) the remainder was used for KRAS evaluation, without a microscope evaluation thereof.

Although it has been documented that carcinoma of the pancreas is frequently associated with KRAS gene mutations, there are reported experiences where KRAS mutation is detected also in patients suffering from chronic pancreatitis. This type of results stems from the use of techniques of exclusively qualitative type, not enabling to quantify mutation levels present in the sample under examination.

The present invention allows to solve the above-mentioned problems by providing a method and a kit for the diagnosis of ductal carcinoma of the pancreas (PDC) enabling to distinguish between chronic pancreatitis and PDC.

### SUMMARY OF THE INVENTION

The present invention is based on the surprising discovery that the mere presence of mutations in the above-indicated codons of the Kras gene, and therefore their qualitative determination (presence/absence of mutation), is no sufficient instrument for a reliable diagnosis of ductal carcinoma of the pancreas (PDC). On the contrary, diagnosis reliability depends on a quantitative determination in terms of percentage of cells bearing Kras gene mutations. Therefore, the present invention is based on the selection of a technique sufficiently sensitive for the determination of the percentage of mutated cells, and on the identification of a percentage value threshold capable of discriminating between chronic pancreatitis and PDC.

The solution to this problem is therefore a diagnostic method enabling the quantitative determination of mutations of the Kras gene by a suitable technology, such as, e.g., pyrosequencing, in a cytological sample obtained by endoscopic ultrasound-guided fine-needle aspiration biopsy.

Therefore, a first object of the present invention is an *in vitro* method for diagnosing the presence of ductal carcinoma of the pancreas in a patient from a patient with solid lesion of the pancreas, comprising the following steps:
a) preparing a cytological sample of said patient obtained by endoscopic ultrasound-guided fine-needle aspiration biopsy;
b) determining by pyrosequencing the percentage of mutation in at least one of the codons 12,13, 61 or 146 of the Kras gene in said sample;
c) diagnosing the presence of ductal carcinoma of the pancreas and/or pancreatitis in said patient by comparing the percentage of mutation obtained in step b) with a threshold value (or cut-off).

In an embodiment of the invention the diagnosis is performed on a sample selected beforehand by cytological analysis techniques.

A second object is the use of a kit comprising means apt to determine in a cell sample the percentage of mutation in codons 12,13, 61 and/or 146 of the Kras gene for diagnosing the presence of ductal carcinoma of the pancreas and/or pancreatitis in a patient.

In an embodiment of the invention, the kit comprises, beside the means apt to determine the percentage of mutation, instructions that indicate a reference percentage above which the presence of ductal carcinoma of the pancreas is diagnosed.

The method of the invention offers the advantage of a diagnosis with a lower incidence of false-positive or false-negative answers. Further advantages, as well as the features and the operation steps of the present invention will be made apparent in the following detailed description of some preferred embodiments thereof.

### DETAILED DESCRIPTION OF THE INVENTION

The present invention consists of the following aspects, described in detail below.

### Sample to be analyzed

The biological sample subjected to analysis is represented by cells from pancreatic lesions, obtained by endoscopic ultrasound-guided fine-needle aspiration biopsy (EUS-FNA) from a patient with solid lesion of the pancreas. The sample to be analyzed could be fresh (i.e., just prepared), frozen or fixed in suitable solutions , like, e.g., alcoholic solutions, formalin and paraformaldehyde, to prevent the biological tissue from degrading.

In particular, cytological material smeared (cytological smear) on slides could be used, set up according to the "thin layer" method or coming from cytological inclusions (cell-blocks). By "cytological smear" it is meant a preparation obtained by smearing on a microscope slide the material collected by thin needle. Said preparation will subsequently be placed in alcoholic solutions or treated with specific sprays for ensuring adequate preservation of the biological material (fixation). By "setting up according to the thin layer method" it is meant a preparation obtained by fixing in alcoholic solutions the material collected by thin needle; the material is subsequently subjected to microfiltration and then transferred onto a microscope slide. By "cytoincluded" it is meant a preparation obtained by immediately fixing in alcoholic solutions the material collected by fine needle and subsequently processed in order to obtain an inclusion in paraffin. From the inclusion in paraffin, sections will be set up for microscope analysis.

In an embodiment of the invention, the diagnosis is performed on a sample selected beforehand by cytological analysis techniques. Cytological analysis is performed by observing the biological sample, suitably treated as described above, through use of an optical microscope. Microscope analysis enables the pathologist to determine the presence and amount of cells and evaluate any morphostructural modifications (cytological atypicalities) thereof.

Cytological analysis could provide a microscopic classification of the cytological material in which, e.g., the slides set up (smeared, set up according to the thin layer method or coming from cytoinclusion) are microscopically evaluated by qualified medical staff, who formulate the cytological classification of the sample according to the following diagnostic classes:
Class 1. sample without cells;
Class 2. sample containing normal pancreatic cells;
Class 3. sample containing a conspicuous share of "atypical" cells, such that the sole cytological analysis does not enable to discriminate whether the sample is an adenocarcinoma or chronic pancreatitis.
Class 4. sample containing few cells, certainly of adenocarcinoma.
Class 5. sample containing numerous cells, certainly of adenocarcinoma.

Preparations morphologically classified in Classes 1 and 2 are to be considered as non-diagnostic, as there is no evidence significant pathological material has been collected. Preparations classified in Classes 4 and 5 are to be considered as diagnostic, as there is clear evidence that the lesions are referable to PDC.

Preparations classified in Class 3 represent the diagnostic dilemma subject of the present invention. In fact, the sole cytomorphological evaluation does not enable a clear diagnostic assignment.

The morphological classification proposed will enable to select the cases in which analysis of KRAS mutations is advisable, discarding cases in which KRAS analysis would be useless or superfluous. Such mutational analysis should be performed on the same material used for cytological diagnosis.

To be considered suitable for analysis, the cytological material should have the following characteristics: a) the share of "atypical" cells should be conspicuous (at least 100 cells), b) the share of atypical cells should be sufficiently represented (at least 50% of the total material should consist of cells with cytological atypicalities).

In a good percentage of cases, criterion b) might not be met as the collected material also contains a share of normal cell laminas. In these cases, this can be obviated by performing an "enrichment" of the pathological material through (manual or laser-assisted) microdissection of the sample, so as to exclude normal cells from the analysis. Said procedure can be performed manually by qualified medical staff, on smeared slides or cytoinclusion-deriving sections.

Thus, false-negative cases are reduced, overall determining an increase in the Test specificity. The cases in which it will be useful to proceed to an examination of Kras gene mutations will preferably be those in which morphological analysis is unable to distinguish pancreatitis from pancreas tumour.

### Kras gene

Human KRAS gene is located on chromosome 12. Its longer isoform (transcription variant A) has 5436 bases and is present in the GenBank database with the sequence identifier NM_033360. The predominant isoform of the Kras gene (transcription variant B) has 5312 bases and is present in the GenBank database with the sequence identifier NM_004985.

The nucleotide sequence of transcription variant A corresponds to SEQ ID 1, whereas transcription variant B corresponds to nucleotide sequence SEQ ID 2. In the present description, the term "mutation" refers to changes in the sequence of base pairs (bp) of the genetic material. Examples of mutations are insertions, where one or more additional bases are added into the bp sequence, deletions, where one or more bases are removed from the bp sequence, and substitutions, in which one or more bases are exchanged. The term "mutation" can also refer to alterations, like, e.g., chromosome changes translocations and inversions.

Examples of known mutations in the codon 12 or 13 of exon 2 of the Kras gene are reported below:
G12A 35 g<c
G12C 34-36 ggt>tgc
G12D 35 g>a
G12R 34 g>c
G12S 34 g>a
G12V 35-36 gt>tc
G12L 34-35 gg>cc
G12N 34-35 gg>aa
G12E 35-36 gt>aa
G12F 34-35 tt
G12G 36 t>a
G12Y 34-35 gg>ta
G13A 38 g>c
G13C 37 g>t
G13D 38-39 gc>at
G13G 39 c>g
G131 37-38 gg>at
G13R 37-39 ggc>cgt
G13S 37 g>a
G13V 38-39 gc>tg

Examples of known mutations in the codon 61 of exon 3 of the Kras gene are reported below:
Q61 E 181 c>g
Q61 H 183 a>t
Q61 K 181 c>a
Q61 L 182 a>t
Q61 P 182 a>c
Q61 R 182 a>g

Examples of known mutations in the codon 146 of the Kras gene are reported below:
A146T 436g>a
A146P 436g>c
A146V 437c>t

### Determining mutations by pyrosequencing.

The method of the present invention provides the determination of the presence or absence of at least one mutation in at least one of the codons 12, 13, 61 or 146 of the Kras gene in the sample to be analyzed by pyrosequencing.

Pyrosequencing is a gene sequencing method entailing numerous advantages with respect to standard sequencing techniques (direct sequencing or Sanger). In particular, this technique is extremely sensitive, enabling to determine mutation levels even when present in 5% of analyzed cells. Moreover, it enables quantitation of the percentage of mutation present in the sample under examination. In the present description, the term "pyrosequencing" refers to a nucleic acid sequencing method based on a "synthesis sequence", in which a single strand of DNA to be sequenced is used, and its complementary strand is synthesized enzymatically. Pyrosequencing is based on detection of pyrophosphate released at nucleotide incorporation. The method enables the sequencing of a single strand of DNA by synthesizing its complementary strand, one base pair at a time, and detecting which base has actually been added at each step. The principle and the basic application of the pyrosequencing technique are described in the publication by Fakhrai-Rad et al. (Hum Mutat. 2002;19:479-485) which is incorporated herein by reference.

The pyrosequencing method can be fundamentally described in four steps:
1. Single-stranded DNA is hybridized to a pyrosequencing primer and incubated with the enzymes, DNA polymerase, ATP sulfurylase, luciferase, and apyrase as well as the substrates adenosine 5' phosphosulfate (APS) and luciferin.
2. One of the four deoxynucleotide triphosphates (dNTP) is added to the reaction mixture of 1. to start the second step, in which DNA polymerase catalyzes the incorporation of the deoxynucleotide triphosphate into the DNA strand, if it is complementary to the base in the template strand. Each incorporation event is accompanied by release of pyrophosphate (PPi) in a quantity equimolar to the amount of incorporated nucleotide.
3. ATP sulfurylase quantitatively converts PPi to ATP in the presence of adenosine 5' phosphosulfate. This ATP in turn acts as substrate for the luciferase-mediated conversion of luciferin to oxyluciferin that generates visible light in amounts that are proportional to the amount of ATP. The light produced in the luciferase reaction is detected by suitable instruments. The intensity of each peak is proportional to the number of nucleotides incorporated.
4. Unincorporated nucleotides and ATP are degraded by apyrase, and the reaction can start again with another nucleotide.

Generally, prior to the pyrosequencing step, a region comprising the part of the gene in which there mutations to be detected can be present is amplified by means of a nucleic acid amplification technique, like polymerase chain reaction (PCR) or variants thereof.

The method could therefore provide a preliminary step of amplification by PCR of the region of DNA of the gene Kras comprising the codon 12, 13, 61 or 146. All PCR variants known in the state of the art could be used, e.g. allel-specific PCR, PCR assembly, asymmetric PCR, Inverse PCR, ligation-mediated PCR, multiplex ligation PCR, nested PCR, quantitative PCR, RT-PCR, TAIL-PCR, etc.

In an embodiment, the determination of the percentage of mutation by pyrosequencing could be carried out as described in European Patent Application EP 2 327 793 A1 or in the publication of Ogino et al. (J Mol Diagn. 2005;7:413-421), both incorporated herein by reference.

The term "primer for pyrosequencing" in the present description denotes a primer suitable for starting the pyrosequencing reaction. The sequence of the pyrosequencing primer will preferably be selected so as to lie at a short distance from the 5' end of the mutation, in order to facilitate the pyrosequencing process. In the method described herein, one or more primers for pyrosequencing could be used. E.g., for the pyrosequencing of codon 12 one or more of the primers as reported in Table 1 could be used. E.g., SEQ ID NO:3 will be used as forward primer in the amplification step, SEQ ID NO:4 will be used as reverse primer, whereas SEQ ID NO:5 will be used as sequencing primer.

The wording "determining the percentage of mutation in at least one of codons 12, 13, 61 or 146 of the gene Kras in the cells of said sample" indicates that in the pyrosequencing step of the method the percentage of mutation will be determined in only one of the codons selected from the group consisting of codon 12, 13, 61 or 146 of the gene Kras, or in two, three or in all four of the codons.

### Diagnosing the presence of ductal carcinoma of the pancreas

Upon determining the percentage of mutation in the sample as described above, the method provides a step wherein the percentage is compared with a reference value. If the percentage of mutation is greater then the reference value, the presence of a pancreas cancer could be diagnosed in the patient from which the sample was collected, whereas, if the percentage is lower than the reference value and the case history suggested a pancreatitis, the diagnosis of pancreatitis could be confirmed.

The reference value will be comprised between 5% and 7%, for instance about 6%, but could also be, e.g., a value obtained by analyzing by pyrosequencing a suitably selected control sample.

### KIT

An *in vitro* kit for diagnosing, comprising means apt to determine in a cell sample the percentage of mutation in codons 12,13, 61 or 146 of the gene Kras and instructions that indicate a reference percentage of mutation above which it is diagnosed the presence of ductal carcinoma of the pancreas.

In an embodiment of the invention, the kit comprises, beside the means apt to determine the percentage of mutations, instructions that indicate a reference percentage above which it is diagnosed the presence of ductal carcinoma of the pancreas. Said percentage for codons 12,13,61 and 146 will be about 6%.

Purely by way of example, the kit could be comprised of the same reagents present in the kit commercially available with the name Anti-EFGR MoAb response^{®} KIT (in the state of the art this kit is used for a completely different purpose, i.e. the prediction of colon cancer patients' response to antibody therapy), which however should be integrated with instructions that indicate the percentage of mutation in the codons 12, 13, 61 or 146, above which tumour presence could be diagnosed, e.g. indicating a percentage of 6%.

The present invention has hereto been described with reference to some embodiments thereof. It is understood that other embodiments might exist, all falling within the concept of the same invention, and all comprised within the protective scope of the claims hereinafter.

### EXAMPLES 1

### Materials

Extraction, amplification and pyrosequencing of samples was performed with the reagents, instruments and controls contained in the Anti-EFGR MoAb response^{®} KIT, marketed in Italy by Diatech Pharmacogenetics Company with Catalogue number UP032 and described in the user manual. The use of this kit gave the results set forth below.

### Patients selection

A) Test validation on histological case histories. 10 cases of chronic pancreatitis not associated to neoplasia and 27 cases of pancreatic carcinoma were selected. In the latter cases, Kras analysis was performed both on tumour tissue and non-neoplastic tissue (at least 1 cm away from the neoplasia), affected by chronic phlogosis phenomena (pancreatitis accompanying the neoplasia).
B) Evaluation on cytological samples deriving from FNA of solid lesions of the pancreas. There were evaluated the cytological cases that had proved doubtful to morphological examination (slight or moderate epithelial atipicality) and which subsequently documented pancreatic carcinoma (12 cases) (histologically or clinically) and chronic pancreatitis in the absence of neoplasia (15 cases) (histologically or clinically).

General Criteria of patient inclusion:
Patients subjected to pancreaticoduodenectomy.
Patients suffering from chronic pancreatitis and/or ductal carcinoma of the pancreas.
Patients without anti-neoplastic treatment under neoadjuvant chemo- and/or radiotherapic regimen.
Patients without a previous history of other neoplastic pathology.
Analysis of Kras mutation in codons 12, 13, 61

For the mutational analysis of Kras codons 12, 13, 61, Anti-EGFR MoAb response Kit, based on the Pyrosequencing technique, was utilized. In the qualitative evaluation, the method proposed enabled the analysis of the allele frequency of said mutation (quantitative evaluation). This latter data helped to define a diagnostic cut-off, with the aim of obtaining a 100% test specificity in singling out PDC on cytological material.

Moreover, always by utilizing the same kit, the study was extended to codon 146 mutations, for which in the literature there are no data in connection with a possible involvement thereof in the tumour pathology in the pancreas.

### Results

In the histological samples, statistically significant differences in terms of percentage of mutation in the Kras gene between the PDCs and the pancreatic tissue site of chronic pancreatitis (p= 0.0001) were detected. KRAS mutation was detected for codons 12 (91.6%), 13 (4.2%) and 61 (4.2%). Codon 146 proved constantly non-mutated. These results are in line with what is expressed up to now by scientific data available to date. In fact, although in human solid tumours KRAS mutations can be detected in codons 12, 13, 61 and 146, as to the carcinoma of the pancreas, KRAS mutations are nearly exclusively detected on codon 12 (98.9%). For completeness' sake, it is reported that mutations in codon 13 and 61 represent the 0.8% and 0.3%, respectively, of KRAS mutations detected in pancreatic carcinoma. It is specified that the allele frequency value equal to 5% represents the "resolution limit" of the pyrosequencing method (Ogino et al. J Mol Diagn. 2005;7:413-421). In other words, below the 5% value the method does not ensure a correct determination of the presence of mutation. In line with what was mentioned above, the preliminary results obtained document that the pancreatic carcinomas examined show an allele frequency of mutation always above 7%, whereas non-neoplastic tissues (e.g., chronic pancreatitis) show a percentage of mutation always below 5.6%. For this reason, an allele frequency value greater than 6% ensures adequate sensitivity and specificity of the test.

In particular, the allele frequency of KRAS mutation proved to be: in PDC samples, of 24.9% (median value) (range: 0.7 - 78.6%; interquartile range (IQR): 13.6 - 32.1 %); in the tissue affected by chronic pancreatitis, of 0.65% (median value) (range: 0 - 5%; IQR: 0.07 - 1.9%); in samples of pancreatitis of patients with PDC diagnosis, of 3.70% (median value) (range: 0.5 - 5.6%; IQR: 2.15 - 4.35%).

On these bases, the allele frequency of mutation of 6% was defined as cut-off for distinguishing inflammatory lesions (chronic pancreatitis) from PDC.

As to the cytological samples deriving from FNA, KRAS mutation analysis was carried out successfully, both in terms of quantity and quality of extracted DNA, in all samples under examination. KRAS mutation allele frequency was detected in a range comprised between 0 and 31.6%; in no case an allele frequency of mutation greater than 6% was detected in cytological samples with a definitive diagnosis of pancreatitis; on the contrary, 11/12 needle aspirates with a definitive diagnosis of carcinoma showed an allele frequency of mutation greater than 6%.

The allele frequency value equal to 5% represents the "resolution limit" of the pyrosequencing method. In other words, below the 5% value the method does not ensure a correct determination of the presence of mutation. In line with what was mentioned above, the preliminary results obtained document that the examined pancreatic carcinomas show an allele frequency of mutation always above 7%, whereas non-neoplastic tissues (e.g., chronic pancreatitis) show a percentage of mutation always below 5.6%. For this reason, an allele frequency value greater than 6% ensures adequate sensitivity and specificity of the test.

### Conclusions

Data obtained to date suggest that analysis of KRAS mutation by the pyrosequencing method is of extreme clinical utility in early diagnosis of the adenocarcinoma of the pancreas, in particular in cases in which a morphological study does not enable a definitive diagnostic conclusion.

### SEQUENCE DESCRIPTION

SEQ ID NO: 1 NM_033360
1 ggccgcggcg gcggaggcag cagcggcggc ggcagtggcg gcggcgaagg tggcggcggc
61 tcggccagta ctcccggccc ccgccatttc ggactgggag cgagcgcggc gcaggcactg
121 aaggcggcgg cggggccaga ggctcagcgg ctcccaggtg cgggagagag gcctgctgaa
181 aatgactgaa tataaacttg tggtagttgg agctggtggc gtaggcaaga gtgccttgac
241 gatacagcta attcagaatc attttgtgga cgaatatgat ccaacaatag aggattccta
301 caggaagcaa gtagtaattg atggagaaac ctgtctcttg gatattctcg acacagcagg
361 tcaagaggag tacagtgcaa tgagggacca gtacatgagg actggggagg gctttctttg
421 tgtatttgcc ataaataata ctaaatcatt tgaagatatt caccattata gagaacaaat
481 taaaagagtt aaggactctg aagatgtacc tatggtccta gtaggaaata aatgtgattt
541 gccttctaga acagtagaca caaaacaggc tcaggactta gcaagaagtt atggaattcc
601 ttttattgaa acatcagcaa agacaagaca gagagtggag gatgcttttt atacattggt
661 gagggagatc cgacaataca gattgaaaaa aatcagcaaa gaagaaaaga ctcctggctg
721 tgtgaaaatt aaaaaatgca ttataatgta atctgggtgt tgatgatgcc ttctatacat
781 tagttcgaga aattcgaaaa cataaagaaa agatgagcaa agatggtaaa aagaagaaaa
841 agaagtcaaa gacaaagtgt gtaattatgt aaatacaatt tgtacttttt tcttaaggca
901 tactagtaca agtggtaatt tttgtacatt acactaaatt attagcattt gttttagcat
961 tacctaattt ttttcctgct ccatgcagac tgttagcttt taccttaaat gcttatttta
1021 aaatgacagt ggaagttttt ttttcctcta agtgccagta ttcccagagt tttggttttt
1081 gaactagcaa tgcctgtgaa aaagaaactg aatacctaag atttctgtct tggggttttt
1141 ggtgcatgca gttgattact tcttattttt cttaccaatt gtgaatgttg gtgtgaaaca
1201 aattaatgaa gcttttgaat catccctatt ctgtgtttta tctagtcaca taaatggatt
1261 aattactaat ttcagttgag accttctaat tggtttttac tgaaacattg agggaacaca
1321 aatttatggg cttcctgatg atgattcttc taggcatcat gtcctatagt ttgtcatccc
1381 tgatgaatgt aaagttacac tgttcacaaa ggttttgtct cctttccact gctattagtc
1441 atggtcactc tccccaaaat attatatttt ttctataaaa agaaaaaaat ggaaaaaaat
1501 tacaaggcaa tggaaactat tataaggcca tttccttttc acattagata aattactata
1561 aagactccta atagcttttc ctgttaaggc agacccagta tgaaatgggg attattatag
1621 caaccatttt ggggctatat ttacatgcta ctaaattttt ataataattg aaaagatttt
1681 aacaagtata aaaaattctc ataggaatta aatgtagtct ccctgtgtca gactgctctt
1741 tcatagtata actttaaatc ttttcttcaa cttgagtctt tgaagatagt tttaattctg
1801 cttgtgacat taaaagatta tttgggccag ttatagctta ttaggtgttg aagagaccaa
1861 ggttgcaagg ccaggccctg tgtgaacctt tgagctttca tagagagttt cacagcatgg
1921 actgtgtccc cacggtcatc cagtgttgtc atgcattggt tagtcaaaat ggggagggac
1981 tagggcagtt tggatagctc aacaagatac aatctcactc tgtggtggtc ctgctgacaa
2041 atcaagagca ttgcttttgt ttcttaagaa aacaaactct tttttaaaaa ttacttttaa
2101 atattaactc aaaagttgag attttggggt ggtggtgtgc caagacatta attttttttt
2161 taaacaatga agtgaaaaag ttttacaatc tctaggtttg gctagttctc ttaacactgg
2221 ttaaattaac attgcataaa cacttttcaa gtctgatcca tatttaataa tgctttaaaa
2281 taaaaataaa aacaatcctt ttgataaatt taaaatgtta cttattttaa aataaatgaa
2341 gtgagatggc atggtgaggt gaaagtatca ctggactagg aagaaggtga cttaggttct
2401 agataggtgt cttttaggac tctgattttg aggacatcac ttactatcca tttcttcatg
2461 ttaaaagaag tcatctcaaa ctcttagttt ttttttttta caactatgta atttatattc
2521 catttacata aggatacact tatttgtcaa gctcagcaca atctgtaaat tttaacctta
2581 tgttacacca tcttcagtgc cagtcttggg caaaattgtg caagaggtga agtttatatt
2641 tgaatatcca ttctcgtttt aggactcttc ttccatatta gtgtcatctt gcctccctac
2701 cttccacatg ccccatgact tgatgcagtt ttaatacttg taattcccct aaccataaga
2761 tttactgctg ctgtggatat ctccatgaag ttttcccact gagtcacatc agaaatgccc
2821 tacatcttat ttcctcaggg ctcaagagaa tctgacagat accataaagg gatttgacct
2881 aatcactaat tttcaggtgg tggctgatgc tttgaacatc tctttgctgc ccaatccatt
2941 agcgacagta ggatttttca aacctggtat gaatagacag aaccctatcc agtggaagga
3001 gaatttaata aagatagtgc tgaaagaatt ccttaggtaa tctataacta ggactactcc
3061 tggtaacagt aatacattcc attgttttag taaccagaaa tcttcatgca atgaaaaata
3121 ctttaattca tgaagcttac tttttttttt tggtgtcaga gtctcgctct tgtcacccag
3181 gctggaatgc agtggcgcca tctcagctca ctgcaacctc catctcccag gttcaagcga
3241 ttctcgtgcc tcggcctcct gagtagctgg gattacaggc gtgtgccact acactcaact
3301 aatttttgta tttttaggag agacggggtt tcaccctgtt ggccaggctg gtctcgaact
3361 cctgacctca agtgattcac ccaccttggc ctcataaacc tgttttgcag aactcattta
3421 ttcagcaaat atttattgag tgcctaccag atgccagtca ccgcacaagg cactgggtat
3481 atggtatccc caaacaagag acataatccc ggtccttagg tagtgctagt gtggtctgta
3541 atatcttact aaggcctttg gtatacgacc cagagataac acgatgcgta ttttagtttt
3601 gcaaagaagg ggtttggtct ctgtgccagc tctataattg ttttgctacg attccactga
3661 aactcttcga tcaagctact ttatgtaaat cacttcattg ttttaaagga ataaacttga
3721 ttatattgtt tttttatttg gcataactgt gattctttta ggacaattac tgtacacatt
3781 aaggtgtatg tcagatattc atattgaccc aaatgtgtaa tattccagtt ttctctgcat
3841 aagtaattaa aatatactta aaaattaata gttttatctg ggtacaaata aacaggtgcc
3901 tgaactagtt cacagacaag gaaacttcta tgtaaaaatc actatgattt ctgaattgct
3961 atgtgaaact acagatcttt ggaacactgt ttaggtaggg tgttaagact tacacagtac
4021 ctcgtttcta cacagagaaa gaaatggcca tacttcagga actgcagtgc ttatgagggg
4081 atatttaggc ctcttgaatt tttgatgtag atgggcattt ttttaaggta gtggttaatt
4141 acctttatgt gaactttgaa tggtttaaca aaagatttgt ttttgtagag attttaaagg
4201 gggagaattc tagaaataaa tgttacctaa ttattacagc cttaaagaca aaaatccttg
4261 ttgaagtttt tttaaaaaaa gctaaattac atagacttag gcattaacat gtttgtggaa
4321 gaatatagca gacgtatatt gtatcatttg agtgaatgtt cccaagtagg cattctaggc

4381 tctatttaac tgagtcacac tgcataggaa tttagaacct aacttttata ggttatcaaa
4441 actgttgtca ccattgcaca attttgtcct aatatataca tagaaacttt gtggggcatg
4501 ttaagttaca gtttgcacaa gttcatctca tttgtattcc attgattttt tttttcttct
4561 aaacattttt tcttcaaaca gtatataact ttttttaggg gatttttttt tagacagcaa
4621 aaactatctg aagatttcca tttgtcaaaa agtaatgatt tcttgataat tgtgtagtaa
4681 tgttttttag aacccagcag ttaccttaaa gctgaattta tatttagtaa cttctgtgtt
4741 aatactggat agcatgaatt ctgcattgag aaactgaata gctgtcataa aatgaaactt
4801 tctttctaaa gaaagatact cacatgagtt cttgaagaat agtcataact agattaagat
4861 ctgtgtttta gtttaatagt ttgaagtgcc tgtttgggat aatgataggt aatttagatg
4921 aatttagggg aaaaaaaagt tatctgcaga tatgttgagg gcccatctct ccccccacac
4981 ccccacagag ctaactgggt tacagtgttt tatccgaaag tttccaattc cactgtcttg
5041 tgttttcatg ttgaaaatac ttttgcattt ttcctttgag tgccaatttc ttactagtac
5101 tatttcttaa tgtaacatgt ttacctggaa tgtattttaa ctatttttgt atagtgtaaa
5161 ctgaaacatg cacattttgt acattgtgct ttcttttgtg ggacatatgc agtgtgatcc
5221 agttgttttc catcatttgg ttgcgctgac ctaggaatgt tggtcatatc aaacattaaa
5281 aatgaccact cttttaattg aaattaactt ttaaatgttt ataggagtat gtgctgtgaa
5341 gtgatctaaa atttgtaata tttttgtcat gaactgtact actcctaatt attgtaatgt
5401 aataaaaata gttacagtga caaaaaaaaa aaaaaa

SEQ ID 2: NM_004985
1 ggccgcggcg gcggaggcag cagcggcggc ggcagtggcg gcggcgaagg tggcggcggc
61 tcggccagta ctcccggccc ccgccatttc ggactgggag cgagcgcggc gcaggcactg
121 aaggcggcgg cggggccaga ggctcagcgg ctcccaggtg cgggagagag gcctgctgaa
181 aatgactgaa tataaacttg tggtagttgg agctggtggc gtaggcaaga gtgccttgac
241 gatacagcta attcagaatc attttgtgga cgaatatgat ccaacaatag aggattccta
301 caggaagcaa gtagtaattg atggagaaac ctgtctcttg gatattctcg acacagcagg
361 tcaagaggag tacagtgcaa tgagggacca gtacatgagg actggggagg gctttctttg
421 tgtatttgcc ataaataata ctaaatcatt tgaagatatt caccattata gagaacaaat
481 taaaagagtt aaggactctg aagatgtacc tatggtccta gtaggaaata aatgtgattt
541 gccttctaga acagtagaca caaaacaggc tcaggactta gcaagaagtt atggaattcc
601 ttttattgaa acatcagcaa agacaagaca gggtgttgat gatgccttct atacattagt
661 tcgagaaatt cgaaaacata aagaaaagat gagcaaagat ggtaaaaaga agaaaaagaa
721 gtcaaagaca aagtgtgtaa ttatgtaaat acaatttgta cttttttctt aaggcatact
781 agtacaagtg gtaatttttg tacattacac taaattatta gcatttgttt tagcattacc
841 taattttttt cctgctccat gcagactgtt agcttttacc ttaaatgctt attttaaaat
901 gacagtggaa gttttttttt cctctaagtg ccagtattcc cagagttttg gtttttgaac
961 tagcaatgcc tgtgaaaaag aaactgaata cctaagattt ctgtcttggg gtttttggtg
1021 catgcagttg attacttctt atttttctta ccaattgtga atgttggtgt gaaacaaatt
1081 aatgaagctt ttgaatcatc cctattctgt gttttatcta gtcacataaa tggattaatt
1141 actaatttca gttgagacct tctaattggt ttttactgaa acattgaggg aacacaaatt
1201 tatgggcttc ctgatgatga ttcttctagg catcatgtcc tatagtttgt catccctgat
1261 gaatgtaaag ttacactgtt cacaaaggtt ttgtctcctt tccactgcta ttagtcatgg
1321 tcactctccc caaaatatta tattttttct ataaaaagaa aaaaatggaa aaaaattaca
1381 aggcaatgga aactattata aggccatttc cttttcacat tagataaatt actataaaga
1441 ctcctaatag cttttcctgt taaggcagac ccagtatgaa atggggatta ttatagcaac
1501 cattttgggg ctatatttac atgctactaa atttttataa taattgaaaa gattttaaca
1561 agtataaaaa attctcatag gaattaaatg tagtctccct gtgtcagact gctctttcat
1621 agtataactt taaatctttt cttcaacttg agtctttgaa gatagtttta attctgcttg
1681 tgacattaaa agattatttg ggccagttat agcttattag gtgttgaaga gaccaaggtt
1741 gcaaggccag gccctgtgtg aacctttgag ctttcataga gagtttcaca gcatggactg
1801 tgtccccacg gtcatccagt gttgtcatgc attggttagt caaaatgggg agggactagg
1861 gcagtttgga tagctcaaca agatacaatc tcactctgtg gtggtcctgc tgacaaatca
1921 agagcattgc ttttgtttct taagaaaaca aactcttttt taaaaattac ttttaaatat
1981 taactcaaaa gttgagattt tggggtggtg gtgtgccaag acattaattt tttttttaaa
2041 caatgaagtg aaaaagtttt acaatctcta ggtttggcta gttctcttaa cactggttaa
2101 attaacattg cataaacact tttcaagtct gatccatatt taataatgct ttaaaataaa
2161 aataaaaaca atccttttga taaatttaaa atgttactta ttttaaaata aatgaagtga
2221 gatggcatgg tgaggtgaaa gtatcactgg actaggaaga aggtgactta ggttctagat
2281 aggtgtcttt taggactctg attttgagga catcacttac tatccatttc ttcatgttaa
2341 aagaagtcat ctcaaactct tagttttttt tttttacaac tatgtaattt atattccatt
2401 tacataagga tacacttatt tgtcaagctc agcacaatct gtaaattttt aacctatgtt
2461 acaccatctt cagtgccagt cttgggcaaa attgtgcaag aggtgaagtt tatatttgaa
2521 tatccattct cgttttagga ctcttcttcc atattagtgt catcttgcct ccctaccttc
2581 cacatgcccc atgacttgat gcagttttaa tacttgtaat tcccctaacc ataagattta
2641 ctgctgctgt ggatatctcc atgaagtttt cccactgagt cacatcagaa atgccctaca
2701 tcttatttcc tcagggctca agagaatctg acagatacca taaagggatt tgacctaatc
2761 actaattttc aggtggtggc tgatgctttg aacatctctt tgctgcccaa tccattagcg
2821 acagtaggat ttttcaaacc tggtatgaat agacagaacc ctatccagtg gaaggagaat
2881 ttaataaaga tagtgctgaa agaattcctt aggtaatcta taactaggac tactcctggt
2941 aacagtaata cattccattg ttttagtaac cagaaatctt catgcaatga aaaatacttt
3001 aattcatgaa gcttactttt tttttttggt gtcagagtct cgctcttgtc acccaggctg
3061 gaatgcagtg gcgccatctc agctcactgc aacctccatc tcccaggttc aagcgattct
3121 cgtgcctcgg cctcctgagt agctgggatt acaggcgtgt gccactacac tcaactaatt
3181 tttgtatttt taggagagac ggggtttcac cctgttggcc aggctggtct cgaactcctg
3241 acctcaagtg attcacccac cttggcctca taaacctgtt ttgcagaact catttattca
3301 gcaaatattt attgagtgcc taccagatgc cagtcaccgc acaaggcact gggtatatgg
3361 tatccccaaa caagagacat aatcccggtc cttaggtagt gctagtgtgg tctgtaatat

3421 cttactaagg cctttggtat acgacccaga gataacacga tgcgtatttt agttttgcaa
3481 agaaggggtt tggtctctgt gccagctcta taattgtttt gctacgattc cactgaaact
3541 cttcgatcaa gctactttat gtaaatcact tcattgtttt aaaggaataa acttgattat
3601 attgtttttt tatttggcat aactgtgatt cttttaggac aattactgta cacattaagg
3661 tgtatgtcag atattcatat tgacccaaat gtgtaatatt ccagttttct ctgcataagt
3721 aattaaaata tacttaaaaa ttaatagttt tatctgggta caaataaaca ggtgcctgaa
3781 ctagttcaca gacaaggaaa cttctatgta aaaatcacta tgatttctga attgctatgt
3841 gaaactacag atctttggaa cactgtttag gtagggtgtt aagacttaca cagtacctcg
3901 tttctacaca gagaaagaaa tggccatact tcaggaactg cagtgcttat gaggggatat
3961 ttaggcctct tgaatttttg atgtagatgg gcattttttt aaggtagtgg ttaattacct
4021 ttatgtgaac tttgaatggt ttaacaaaag atttgttttt gtagagattt taaaggggga
4081 gaattctaga aataaatgtt acctaattat tacagcctta aagacaaaaa tccttgttga
4141 agttttttta aaaaaagcta aattacatag acttaggcat taacatgttt gtggaagaat
4201 atagcagacg tatattgtat catttgagtg aatgttccca agtaggcatt ctaggctcta
4261 tttaactgag tcacactgca taggaattta gaacctaact tttataggtt atcaaaactg
4321 ttgtcaccat tgcacaattt tgtcctaata tatacataga aactttgtgg ggcatgttaa
4381 gttacagttt gcacaagttc atctcatttg tattccattg attttttttt tcttctaaac
4441 attttttctt caaacagtat ataacttttt ttaggggatt tttttttaga cagcaaaaac
4501 tatctgaaga tttccatttg tcaaaaagta atgatttctt gataattgtg tagtaatgtt
4561 ttttagaacc cagcagttac cttaaagctg aatttatatt tagtaacttc tgtgttaata
4621 ctggatagca tgaattctgc attgagaaac tgaatagctg tcataaaatg aaactttctt
4681 tctaaagaaa gatactcaca tgagttcttg aagaatagtc ataactagat taagatctgt
4741 gttttagttt aatagtttga agtgcctgtt tgggataatg ataggtaatt tagatgaatt
4801 taggggaaaa aaaagttatc tgcagatatg ttgagggccc atctctcccc ccacaccccc
4861 acagagctaa ctgggttaca gtgttttatc cgaaagtttc caattccact gtcttgtgtt
4921 ttcatgttga aaatactttt gcatttttcc tttgagtgcc aatttcttac tagtactatt
4981 tcttaatgta acatgtttac ctggaatgta ttttaactat ttttgtatag tgtaaactga
5041 aacatgcaca ttttgtacat tgtgctttct tttgtgggac atatgcagtg tgatccagtt
5101 gttttccatc atttggttgc gctgacctag gaatgttggt catatcaaac attaaaaatg
5161 accactcttt taattgaaat taacttttaa atgtttatag gagtatgtgc tgtgaagtga
5221 tctaaaattt gtaatatttt tgtcatgaac tgtactactc ctaattattg taatgtaata
5281 aaaatagtta cagtgacaaa aaaaaaaaaa aa

| **Table 1.** Sequences of primers for codon 12 pyrosequencing | |
|---|---|
| | |
| Primer for (forward) PCR (SEQ ID NO:3) | CTGAATATAAACTTGTGGTAGTTG |
| Primer for (reverse) PCR (SEQ ID NO:4) | TGTATCGTCAAGGCACTCT |
| Primer for sequencing (SEQ ID NO:5) | AAACTTGTGGTAGTTGGA |
| Dispensing order (SEQ ID NO:6) | GCTGACGATGAGTCGTA |

### REFERENCES

- Turner BG, Cizginer S, Agarwal D, et al. Diagnosis of pancreatic neoplasia with EUS and FNA: a report of accuracy. Gastrointest Endosc. 2010;71:91-98.
- Bournet B, Souque A, Senesse P,et al. Endoscopic ultrasound-guided fine-needle aspiration biopsy coupled with KRAS mutation assay to distinguish pancreatic cancer from pseudotumoral chronic pancreatitis. Endoscopy. 2009;41:552-557.
- Tada M, Komatsu Y, Kawabe T, et al. Quantitative analysis of K-ras gene mutation in pancreatic tissue obtained by endoscopic ultrasonography-guided fine needle aspiration: clinical utility for diagnosis of pancreatic tumor. Am J Gastroenterol. 2002;97:2263-2270.
- Loupakis F, Ruzzo A, Cremolini C, et al. KRAS codon 61, 146 and BRAF mutations predict resistance to cetuximab plus irinotecan in KRAS codon 12 and 13 wild-type metastatic colorectal cancer. Br J Cancer. 2009;101:715-721.
- Edkins S, O'Meara S, Parker A, et al. Recurrent KRAS codon 146 mutations in human colorectal cancer. Cancer Biol Ther. 2006;5:928-932
- Fakhrai-Rad H, Pourmand N, Ronaghi M. Pyrosequencing: an accurate detection platform for single nucleotide polymorphisms. Hum Mutat. 2002;19:479-485
- EP 2 327 793 A1, European Patent Application
- Ogino S, Kawasaki T, Brahmandam M, et al. Sensitive sequencing method for KRAS mutation detection by Pyrosequencing. J Mol Diagn. 2005;7:413-421.

## Claims

1. An *in vitro* method for diagnosing the presence of ductal carcinoma of the pancreas in a patient from a patient with solid lesion of the pancreas, comprising the following steps:
a) preparing a cytological sample of said patient obtained by endoscopic ultrasound-guided fine-needle aspiration;
b) determining by pyrosequencing the percentage of mutation in codon 12,13, 61 or 146 of the Kras gene in said sample;
c) diagnosing the presence of ductal carcinoma of the pancreas and/or pancreatitis in said patient by comparing the percentage of mutation obtained in step b) with a reference threshold value (cut-off).

2. The method according to claim 1, wherein said threshold value related to mutations in the codon 12 is about 6%.

3. The method according to claim 1 or 2, comprising a preliminary step to step b) wherein said sample is subjected to cytological analysis to select samples containing atypical cells referable to adenocarcinoma or chronic pancreatitis.

4. The method according to claim 3, wherein step b) is performed on the same sample selected by cytological analysis.

5. The method according to any one of claims 1 to 4, comprising a step of amplification by PCR of the region of DNA of the gene Kras comprising the codon 12,13, 61 or 146.

6. The method according to any one of claims 1 to 5, wherein at least one of the polynucleotide sequences selected from the group consisting of SEQ ID NO:3, SEQ ID NO:4, SEQ ID NO:5, SEQ ID NO:6 is used as a primer of pyrosequencing.

7. The method according to any one of claims 1 to 6 for which a percentage of mutation higher than the reference threshold value (cut-off) indicates the presence of ductal carcinoma of the pancreas.

8. An *in vitro* kit for diagnosing the presence of ductal carcinoma of the pancreas in a patient, comprising means apt to determine in a cell sample the percentage of mutation in at least one of the codons 12,13, 61 or 146 of the gene Kras and instructions that indicate a threshold percentage value above which it is diagnosed the presence of pancreas ductal carcinoma, below which it is diagnosed pancreatitis.

9. The kit according to claim 8, wherein said percentage value of threshold related to codon 12 is about 6%.

10. The kit according to claims 8 or 9, wherein the mutational determination is carried out by pyrosequencing.

11. The kit according to any one of claims 8 to 10, wherein said means comprises pyrosequencing primers selected from the group consisting of the polynucleotide sequences SEQ ID NO:3, SEQ ID NO:4, SEQ ID NO:5, SEQ ID NO:6 and reagents suitable for a reaction of pyrosequencing.

12. Use of a kit comprising means apt to determine in a cell sample the percentage of mutation in codons 12,13, 61 or 146 of the gene Kras for diagnosing the presence of ductal carcinoma of the pancreas or pancreatitis.

13. Use of a kit according to claim 12, wherein said means comprises pyrosequencing primers selected from the group consisting of the polynucleotide sequences SEQ ID NO:3, SEQ ID NO:4, SEQ ID NO:5, SEQ ID NO:6 and reagents suitable for a reaction of pyrosequencing.

14. Use of a kit according to claims 12 or 13, comprising instructions that indicate a threshold percentage value above which it is diagnosed the presence of ductal carcinoma of the pancreas, below which it is diagnosed pancreatitis.

15. Use of a kit according to claim 14, wherein said percentage value of threshold related to codon 12 is about 6%.


SEQUENCE LISTING

<110> Università Campus Bio-Medico di Roma

<120> DIAGNOSTIC METHOD OF PANCREAS CANCER BASED ON THE DETERMINATION OF
MUTATIONS OF K-RAS GENE

<130> BE1176R

<160> 6

<170> BiSSAP 1.0

<210> 1
<211> 5436
<212> DNA
<213> Homo sapiens

<220>
<221> source
<222> 1..5436
<223> /mol_type="DNA"
      /organism="Homo sapiens"

<400> 1
ggccgcggcg gcggaggcag cagcggcggc ggcagtggcg gcggcgaagg tggcggcggc      60

tcggccagta ctcccggccc ccgccatttc ggactgggag cgagcgcggc gcaggcactg     120

aaggcggcgg cggggccaga ggctcagcgg ctcccaggtg cgggagagag gcctgctgaa     180

aatgactgaa tataaacttg tggtagttgg agctggtggc gtaggcaaga gtgccttgac     240

gatacagcta attcagaatc attttgtgga cgaatatgat ccaacaatag aggattccta     300

caggaagcaa gtagtaattg atggagaaac ctgtctcttg gatattctcg acacagcagg     360

tcaagaggag tacagtgcaa tgagggacca gtacatgagg actggggagg gctttctttg     420

tgtatttgcc ataaataata ctaaatcatt tgaagatatt caccattata gagaacaaat     480

taaaagagtt aaggactctg aagatgtacc tatggtccta gtaggaaata aatgtgattt     540

gccttctaga acagtagaca caaaacaggc tcaggactta gcaagaagtt atggaattcc     600

ttttattgaa acatcagcaa agacaagaca gagagtggag gatgcttttt atacattggt     660

gagggagatc cgacaataca gattgaaaaa aatcagcaaa gaagaaaaga ctcctggctg     720

tgtgaaaatt aaaaaatgca ttataatgta atctgggtgt tgatgatgcc ttctatacat     780

tagttcgaga aattcgaaaa cataaagaaa agatgagcaa agatggtaaa aagaagaaaa     840

agaagtcaaa gacaaagtgt gtaattatgt aaatacaatt tgtacttttt tcttaaggca     900

tactagtaca agtggtaatt tttgtacatt acactaaatt attagcattt gttttagcat     960

tacctaattt ttttcctgct ccatgcagac tgttagcttt taccttaaat gcttatttta    1020

aaatgacagt ggaagttttt ttttcctcta agtgccagta ttcccagagt tttggttttt    1080

gaactagcaa tgcctgtgaa aaagaaactg aatacctaag atttctgtct tggggttttt    1140

ggtgcatgca gttgattact tcttattttt cttaccaatt gtgaatgttg gtgtgaaaca 1200

aattaatgaa gcttttgaat catccctatt ctgtgtttta tctagtcaca taaatggatt 1260

aattactaat ttcagttgag accttctaat tggtttttac tgaaacattg agggaacaca 1320

aatttatggg cttcctgatg atgattcttc taggcatcat gtcctatagt ttgtcatccc 1380

tgatgaatgt aaagttacac tgttcacaaa ggttttgtct cctttccact gctattagtc 1440

atggtcactc tccccaaaat attatatttt ttctataaaa agaaaaaaat ggaaaaaaat 1500

tacaaggcaa tggaaactat tataaggcca tttccttttc acattagata aattactata 1560

aagactccta atagcttttc ctgttaaggc agacccagta tgaaatgggg attattatag 1620

caaccatttt ggggctatat ttacatgcta ctaaattttt ataataattg aaaagatttt 1680

aacaagtata aaaaattctc ataggaatta aatgtagtct ccctgtgtca gactgctctt 1740

tcatagtata actttaaatc ttttcttcaa cttgagtctt tgaagatagt tttaattctg 1800

cttgtgacat taaaagatta tttgggccag ttatagctta ttaggtgttg aagagaccaa 1860

ggttgcaagg ccaggccctg tgtgaacctt tgagctttca tagagagttt cacagcatgg 1920

actgtgtccc cacggtcatc cagtgttgtc atgcattggt tagtcaaaat ggggagggac 1980

tagggcagtt tggatagctc aacaagatac aatctcactc tgtggtggtc ctgctgacaa 2040

atcaagagca ttgcttttgt ttcttaagaa aacaaactct tttttaaaaa ttacttttaa 2100

atattaactc aaaagttgag attttggggt ggtggtgtgc caagacatta attttttttt 2160

taaacaatga agtgaaaaag ttttacaatc tctaggtttg gctagttctc ttaacactgg 2220

ttaaattaac attgcataaa cacttttcaa gtctgatcca tatttaataa tgctttaaaa 2280

taaaaataaa aacaatcctt ttgataaatt taaaatgtta cttattttaa aataaatgaa 2340

gtgagatggc atggtgaggt gaaagtatca ctggactagg aagaaggtga cttaggttct 2400

agataggtgt cttttaggac tctgattttg aggacatcac ttactatcca tttcttcatg 2460

ttaaaagaag tcatctcaaa ctcttagttt ttttttttta caactatgta atttatattc 2520

catttacata aggatacact tatttgtcaa gctcagcaca atctgtaaat ttttaaccta 2580

tgttacacca tcttcagtgc cagtcttggg caaaattgtg caagaggtga agtttatatt 2640

tgaatatcca ttctcgtttt aggactcttc ttccatatta gtgtcatctt gcctccctac 2700

cttccacatg ccccatgact tgatgcagtt ttaatacttg taattcccct aaccataaga 2760

tttactgctg ctgtggatat ctccatgaag ttttcccact gagtcacatc agaaatgccc 2820

tacatcttat ttcctcaggg ctcaagagaa tctgacagat accataaagg gatttgacct 2880

aatcactaat tttcaggtgg tggctgatgc tttgaacatc tctttgctgc ccaatccatt 2940

agcgacagta ggatttttca aacctggtat gaatagacag aaccctatcc agtggaagga 3000

gaatttaata aagatagtgc tgaaagaatt ccttaggtaa tctataacta ggactactcc 3060

tggtaacagt aatacattcc attgttttag taaccagaaa tcttcatgca atgaaaaata 3120

ctttaattca tgaagcttac tttttttttt tggtgtcaga gtctcgctct tgtcacccag 3180

gctggaatgc agtggcgcca tctcagctca ctgcaacctc catctcccag gttcaagcga 3240

ttctcgtgcc tcggcctcct gagtagctgg gattacaggc gtgtgccact acactcaact 3300

aatttttgta tttttaggag agacggggtt tcaccctgtt ggccaggctg gtctcgaact 3360

cctgacctca agtgattcac ccaccttggc ctcataaacc tgttttgcag aactcattta 3420

ttcagcaaat atttattgag tgcctaccag atgccagtca ccgcacaagg cactgggtat 3480

atggtatccc caaacaagag acataatccc ggtccttagg tagtgctagt gtggtctgta 3540

atatcttact aaggcctttg gtatacgacc cagagataac acgatgcgta ttttagtttt 3600

gcaaagaagg ggtttggtct ctgtgccagc tctataattg ttttgctacg attccactga 3660

aactcttcga tcaagctact ttatgtaaat cacttcattg ttttaaagga ataaacttga 3720

ttatattgtt tttttatttg gcataactgt gattctttta ggacaattac tgtacacatt 3780

aaggtgtatg tcagatattc atattgaccc aaatgtgtaa tattccagtt ttctctgcat 3840

aagtaattaa aatatactta aaaattaata gttttatctg ggtacaaata aacaggtgcc 3900

tgaactagtt cacagacaag gaaacttcta tgtaaaaatc actatgattt ctgaattgct 3960

atgtgaaact acagatcttt ggaacactgt ttaggtaggg tgttaagact tacacagtac 4020

ctcgtttcta cacagagaaa gaaatggcca tacttcagga actgcagtgc ttatgagggg 4080

atatttaggc ctcttgaatt tttgatgtag atgggcattt ttttaaggta gtggttaatt 4140

acctttatgt gaactttgaa tggtttaaca aaagatttgt ttttgtagag attttaaagg 4200

gggagaattc tagaaataaa tgttacctaa ttattacagc cttaaagaca aaaatccttg 4260

ttgaagtttt tttaaaaaaa gctaaattac atagacttag gcattaacat gtttgtggaa 4320

gaatatagca gacgtatatt gtatcatttg agtgaatgtt cccaagtagg cattctaggc 4380

tctatttaac tgagtcacac tgcataggaa tttagaacct aacttttata ggttatcaaa 4440

actgttgtca ccattgcaca attttgtcct aatatataca tagaaacttt gtggggcatg 4500

ttaagttaca gtttgcacaa gttcatctca tttgtattcc attgattttt tttttcttct 4560

aaacattttt tcttcaaaca gtatataact ttttttaggg gatttttttt tagacagcaa 4620

aaactatctg aagatttcca tttgtcaaaa agtaatgatt tcttgataat tgtgtagtaa 4680

tgttttttag aacccagcag ttaccttaaa gctgaattta tatttagtaa cttctgtgtt 4740

aatactggat agcatgaatt ctgcattgag aaactgaata gctgtcataa aatgaaactt 4800

tctttctaaa gaaagatact cacatgagtt cttgaagaat agtcataact agattaagat 4860

ctgtgtttta gtttaatagt ttgaagtgcc tgtttgggat aatgataggt aatttagatg 4920

aatttagggg aaaaaaaagt tatctgcaga tatgttgagg gcccatctct ccccccacac 4980

ccccacagag ctaactgggt tacagtgttt tatccgaaag tttccaattc cactgtcttg 5040

tgttttcatg ttgaaaatac ttttgcattt ttcctttgag tgccaatttc ttactagtac 5100

tatttcttaa tgtaacatgt ttacctggaa tgtattttaa ctatttttgt atagtgtaaa 5160

ctgaaacatg cacattttgt acattgtgct ttcttttgtg ggacatatgc agtgtgatcc 5220

agttgttttc catcatttgg ttgcgctgac ctaggaatgt tggtcatatc aaacattaaa 5280

aatgaccact cttttaattg aaattaactt ttaaatgttt ataggagtat gtgctgtgaa 5340

gtgatctaaa atttgtaata tttttgtcat gaactgtact actcctaatt attgtaatgt 5400

aataaaaata gttacagtga caaaaaaaaa aaaaaa 5436

<210> 2
<211> 5312
<212> DNA
<213> Homo sapiens

<220>
<221> source
<222> 1..5312
<223> /mol_type="DNA"
      /organism="Homo sapiens"

<400> 2
ggccgcggcg gcggaggcag cagcggcggc ggcagtggcg gcggcgaagg tggcggcggc 60

tcggccagta ctcccggccc ccgccatttc ggactgggag cgagcgcggc gcaggcactg 120

aaggcggcgg cggggccaga ggctcagcgg ctcccaggtg cgggagagag gcctgctgaa 180

aatgactgaa tataaacttg tggtagttgg agctggtggc gtaggcaaga gtgccttgac 240

gatacagcta attcagaatc attttgtgga cgaatatgat ccaacaatag aggattccta 300

caggaagcaa gtagtaattg atggagaaac ctgtctcttg gatattctcg acacagcagg 360

tcaagaggag tacagtgcaa tgagggacca gtacatgagg actggggagg gctttctttg 420

tgtatttgcc ataaataata ctaaatcatt tgaagatatt caccattata gagaacaaat 480

taaaagagtt aaggactctg aagatgtacc tatggtccta gtaggaaata aatgtgattt 540

gccttctaga acagtagaca caaaacaggc tcaggactta gcaagaagtt atggaattcc 600

ttttattgaa acatcagcaa agacaagaca gggtgttgat gatgccttct atacattagt 660

tcgagaaatt cgaaaacata aagaaaagat gagcaaagat ggtaaaaaga agaaaaagaa 720

gtcaaagaca aagtgtgtaa ttatgtaaat acaatttgta cttttttctt aaggcatact 780

agtacaagtg gtaatttttg tacattacac taaattatta gcatttgttt tagcattacc 840

taattttttt cctgctccat gcagactgtt agcttttacc ttaaatgctt attttaaaat 900

gacagtggaa gttttttttt cctctaagtg ccagtattcc cagagttttg gtttttgaac 960

tagcaatgcc tgtgaaaaag aaactgaata cctaagattt ctgtcttggg gtttttggtg 1020

catgcagttg attacttctt atttttctta ccaattgtga atgttggtgt gaaacaaatt 1080

aatgaagctt ttgaatcatc cctattctgt gttttatcta gtcacataaa tggattaatt 1140

actaatttca gttgagacct tctaattggt ttttactgaa acattgaggg aacacaaatt 1200

tatgggcttc ctgatgatga ttcttctagg catcatgtcc tatagtttgt catccctgat 1260

gaatgtaaag ttacactgtt cacaaaggtt ttgtctcctt tccactgcta ttagtcatgg 1320

tcactctccc caaaatatta tattttttct ataaaaagaa aaaaatggaa aaaaattaca 1380

aggcaatgga aactattata aggccatttc cttttcacat tagataaatt actataaaga 1440

ctcctaatag cttttcctgt taaggcagac ccagtatgaa atggggatta ttatagcaac 1500

cattttgggg ctatatttac atgctactaa atttttataa taattgaaaa gattttaaca 1560

agtataaaaa attctcatag gaattaaatg tagtctccct gtgtcagact gctctttcat 1620

agtataactt taaatctttt cttcaacttg agtctttgaa gatagtttta attctgcttg 1680

tgacattaaa agattatttg ggccagttat agcttattag gtgttgaaga gaccaaggtt 1740

gcaaggccag gccctgtgtg aacctttgag ctttcataga gagtttcaca gcatggactg 1800

tgtccccacg gtcatccagt gttgtcatgc attggttagt caaaatgggg agggactagg 1860

gcagtttgga tagctcaaca agatacaatc tcactctgtg gtggtcctgc tgacaaatca 1920

agagcattgc ttttgtttct taagaaaaca aactcttttt taaaaattac ttttaaatat 1980

taactcaaaa gttgagattt tggggtggtg gtgtgccaag acattaattt tttttttaaa 2040

caatgaagtg aaaaagtttt acaatctcta ggtttggcta gttctcttaa cactggttaa 2100

attaacattg cataaacact tttcaagtct gatccatatt taataatgct ttaaaataaa 2160

aataaaaaca atcctttgga taaatttaaa atgttactta ttttaaaata aatgaagtga 2220

gatggcatgg tgaggtgaaa gtatcactgg actaggaaga aggtgactta ggttctagat 2280

aggtgtcttt taggactctg attttgagga catcacttac tatccatttc ttcatgttaa 2340

aagaagtcat ctcaaactct tagttttttt tttttacaac tatgtaattt atattccatt 2400

tacataagga tacacttatt tgtcaagctc agcacaatct gtaaattttt aacctatgtt 2460

acaccatctt cagtgccagt cttgggcaaa attgtgcaag aggtgaagtt tatatttgaa 2520

tatccattct cgttttagga ctcttcttcc atattagtgt catcttgcct ccctaccttc 2580

cacatgcccc atgacttgat gcagttttaa tacttgtaat tcccctaacc ataagattta 2640

ctgctgctgt ggatatctcc atgaagtttt cccactgagt cacatcagaa atgccctaca 2700

tcttatttcc tcagggctca agagaatctg acagatacca taaaggattt tgacctaatc 2760

actaattttc aggtggtggc tgatgctttg aacatctctt tgctgcccaa tccattagcg 2820

acagtaggat ttttcaaacc tggtatgaat agacagaacc ctatccagtg gaaggagaat 2880

ttaataaaga tagtgctgaa agaattcctt aggtaatcta taactaggac tactcctggt 2940

aacagtaata cattccattg ttttagtaac cagaaatctt catgcaatga aaaatacttt 3000

aattcatgaa gcttactttt tttttttggt gtcagagtct cgctcttgtc acccaggctg 3060

gaatgcagtg gcgccatctc agctcactgc aacctccatc tcccaggttc aagcgattct 3120

cgtgcctcgg cctcctgagt agctgggatt acaggcgtgt gccactacac tcaactaatt 3180

tttgtatttt taggagagac ggggtttcac cctgttggcc aggctggtct cgaactcctg 3240

acctcaagtg attcacccac cttggcctca taaacctgtt ttgcagaact catttattca 3300

gcaaatattt attgagtgcc taccagatgc cagtcaccgc acaaggcact gggtatatgg 3360

tatccccaaa caagagacat aatcccggtc cttaggtagt gctagtgtgg tctgtaatat 3420

cttactaagg cctttggtat acgacccaga gataacacga tgcgtatttt agttttgcaa 3480

agaaggggtt tggtctctgt gccagctcta taattgtttt gctacgattc cactgaaact 3540

cttcgatcaa gctactttat gtaaatcact tcattgtttt aaaggaataa acttgattat 3600

attgtttttt tatttggcat aactgtgatt cttttaggac aattactgta cacattaagg 3660

tgtatgtcag atattcatat tgacccaaat gtgtaatatt ccagttttct ctgcataagt 3720

aattaaaata tacttaaaaa ttaatagttt tatctgggta caaataaaca ggtgcctgaa 3780

ctagttcaca gacaaggaaa cttctatgta aaaatcacta tgatttctga attgctatgt 3840

gaaactacag atctttggaa cactgtttag gtagggtgtt aagacttaca cagtacctcg 3900

tttctacaca gagaaagaaa tggccatact tcaggaactg cagtgcttat gaggggatat 3960

ttaggcctct tgaatttttg atgtagatgg gcattttttt aaggtagtgg ttaattacct 4020

ttatgtgaac tttgaatggt ttaacaaaag atttgttttt gtagagattt taaaggggga 4080

gaattctaga aataaatgtt acctaattat tacagcctta aagacaaaaa tccttgttga 4140

agttttttta aaaaaagcta aattacatag acttaggcat taacatgttt gtggaagaat 4200

atagcagacg tatattgtat catttgagtg aatgttccca agtaggcatt ctaggctcta 4260

tttaactgag tcacactgca taggaattta gaacctaact tttataggtt atcaaaactg 4320

ttgtcaccat tgcacaattt tgtcctaata tatacataga aactttgtgg ggcatgttaa 4380

gttacagttt gcacaagttc atctcatttg tattccattg attttttttt tcttctaaac 4440

atttttcttt caaacagtat ataacttttt ttaggggatt tttttttaga cagcaaaaac 4500

tatctgaaga tttccatttg tcaaaaagta atgatttctt gataattgtg tagtaatgtt 4560

ttttagaacc cagcagttac cttaaagctg aatttatatt tagtaacttc tgtgttaata 4620

ctggatagca tgaattctgc attgagaaac tgaatagctg tcataaaatg aaactttctt 4680

tctaaagaaa gatactcaca tgagttcttg aagaatagtc ataactagat taagatctgt 4740

gttttagttt aatagtttga agtgcctgtt tgggataatg ataggtaatt tagatgaatt 4800

taggggaaaa aaaagttatc tgcagatatg ttgagggccc atctctcccc ccacaccccc 4860

acagagctaa ctgggttaca gtgttttatc cgaaagtttc caattccact gtcttgtgtt 4920

ttcatgttga aaatactttt gcatttttcc tttgagtgcc aatttcttac tagtactatt 4980

tcttaatgta acatgtttac ctggaatgta ttttaactat ttttgtatag tgtaaactga 5040

aacatgcaca ttttgtacat tgtgctttct tttgtgggac atatgcagtg tgatccagtt 5100

gttttccatc atttggttgc gctgacctag gaatgttggt catatcaaac attaaaaatg 5160

accactcttt taattgaaat taacttttaa atgtttatag gagtatgtgc tgtgaagtga 5220

tctaaaattt gtaatatttt tgtcatgaac tgtactactc ctaattattg taatgtaata 5280

aaaatagtta cagtgacaaa aaaaaaaaaa aa 5312

<210> 3
<211> 24
<212> DNA
<213> Homo sapiens

<220>
<221> source
<222> 1..24
<223> /mol_type="DNA"
/organism="Homo sapiens"

<400> 3
ctgaatataa acttgtggta gttg 24

<210> 4
<211> 19
<212> DNA
<213> Homo sapiens

<220>
<221> source
<222> 1..19
<223> /mol_type="DNA"
/organism="Homo sapiens"

<400> 4
tgtatcgtca aggcactct 19

<210> 5
<211> 18
<212> DNA
<213> Homo sapiens

<220>
<221> source
<222> 1..18
<223> /mol_type="DNA"
/organism="Homo sapiens"

<400> 5

aaacttgtgg tagttgga                                                    18

<210> 6
<211> 17
<212> DNA
<213> Homo sapiens

<220>
<221> source
<222> 1..17
<223> /mol_type="DNA"
      /organism="Homo sapiens"

<400> 6
gctgacgatg agtcgta                                                     17